# EUROPÄISCHE PATENTSCHRIFT

(11) **EP 1 054 945 B1**
(45) Veröffentlichungstag und Bekanntmachung des Hinweises auf die Patenterteilung: **31.10.2001**
(21) Anmeldenummer: 99910173.6
(22) Anmeldetag: 02.02.1999
(51) Int. Cl.: C11D 1/825, A61K 7/00, B01F 17/34, B01F 17/56

(54) **VERWENDUNG VON DETERGENSGEMISCHEN ALS LÖSUNGSVERMITTLER**
USE OF DETERGENT MIXTURES AS SOLUTIZERS
UTILISATION DE MELANGES DETERSIFS COMME SOLUBILISEURS

(30) Priorität: 11.02.1998 DE 19805433
(43) Veröffentlichungstag der Anmeldung: 29.11.2000
(73) Patentinhaber: Cognis Deutschland GmbH, 40589 Düsseldorf (DE)
(72) Erfinder: ANSMANN, Achim, D-40699 Erkrath (DE); KAWA, Rolf, D-40789 Monheim (DE); TESMANN, Holger, D-41363 Jüchen (DE); BAUMÖLLER, Guido, D-42799 Leichlingen (DE)
(86) Internationale Anmeldenummer: EP9900644
(87) Internationale Veröffentlichungsnummer: WO9941341

(56) Entgegenhaltungen:
- DE-A- 3 830 494
- DE-A- 19 533 539
- PATENT ABSTRACTS OF JAPAN vol. 095, no. 004, 31. Mai 1995 & JP 07 008781 A (TAIYO KAGAKU CO LTD), 13. Januar 1995
- PATENT ABSTRACTS OF JAPAN vol. 098, no. 004, 31. März 1998 & JP 09 315932 A (SHISEIDO CO LTD), 9. Dezember 1997
- WEKEL H -U ET AL: "SOLUBILISIERUNG - EIN UEBERBLICK UEBER EIN FASZINIERENDES PHAENOMEN" SOFW-JOURNAL SEIFEN, OELE, FETTE, WACHSE, Bd. 124, Nr. 11, 1. Oktober 1998, Seiten 744, 746-748, 75, XP000778433

## Beschreibung

### Gebiet der Erfindung

Die Erfindung betrifft die Verwendung von Mischungen aus bestimmten Glykosiden und Polyolestem als Lösungsvermittler für die Herstellung von Solubilisaten.

### Stand der Technik

Lösungsvermittler dienen zur Herstellung von wäßrigen und wäßrig-alkoholischen Pseudolösungen ("Solubilisaten") von wasserunlöslichen Stoffen mit ölähnlichem Verhalten, wie beispielsweise etherischen Ölen oder Vitaminen etc. Das Auflösen dieser Substanzen beruht darauf, daß ihre Löslichkeit in den Tensidmicellen größer ist als in der wäßrigen Phase. Das bedeutet, daß als Lösungsvermittler primär solche Tenside besonders gut geeignet erscheinen, die in der Lage sind, schon bei niedrigen Konzentrationen Micellen zu bilden **[Parf.Kosm. 65, 679 (1984)].** Derartige Tenside enthalten jedoch stets Alkylenoxidketten, was aus Sicht des Marktes nicht immer erwünscht ist.

In diesem Zusammenhang sei darauf hingewiesen, daß die Verwendung von Alkylglucosiden als Emulgatoren für eine Vielzahl von Anwendungen bereits zum Stand der Technik zählt. Die emulgierenden und dispergierenden Eigenschaften der Polyglycerinpoly-12-hydroxystearate ist Gegenstand der beiden deutschen Offenlegungsschriften **DE-A1 4420516** und **DE-A1 19643062.** Mischungen von Alkylglucosiden und Polyglycerinpoly-12-hydroxystearaten und deren Verwendung als Emulgatoren sind in der **DE-A1 19533539** beschrieben. Die Bildung von Emulsionen folgt jedoch einem anderen Mechanismus als die Solubilisierung. Demzufolge kann aus der Eignung eines Stoffes als Emulgator nicht gefolgert werden, daß er auch solubilisierende Eigenschaften besitzt.

Die Aufgabe der vorliegenden Erfindung hat folglich darin bestanden, neue Lösungsvermittler für die Herstellung von Solubilisaten zur Verfügung zu stellen, die kein Alkylenoxid enthalten und im Vergleich mit den Produkten des Stands der Technik wenigstens vergleichbare anwendungstechnische Ergebnisse liefem.

### Beschreibung der Erfindung

Gegenstand der Erfindung ist die Verwendung von Detergensgemischen, enthaltend
(a) Alkyl- und/oder Alkenyloligoglykoside und
(b) Polyolpolyhydroxy-12-stearate
als Lösungsvermittler zur Herstellung von klaren Solubilisaten.

Überraschenderweise wurde gefunden, daß aufeinander abgestimmte Mischungen aus Alkyl- und/oder Alkenyloligoglykosiden und Polyolpolyhydroxystearaten beliebig mit Wasser kalt mischbar sind und daneben auch ausgezeichnete solubilisierende Eigenschaften aufweisen. Somit ist es gemäß der neuen Lehre der Erfindung nunmehr möglich, klare Pseudolösungen mit etherischen Ölen oder Vitaminen zu formulieren, die bisher auf anderem Wege nicht zugänglich waren. Diese Erkenntnis ist auch gerade deswegen überraschend, da die Polyolpolyhydroxystearate zwar grenzflächenaktive Eigenschaften aufweisen, aber dennoch überwiegend lipophilen Charakter besitzen und daher mit Wasser in keinem Verhältnis mischbar sind.

### Alkyl-und/oder Alkenyloligoglykoside

Alkyl- und Alkenyloligoglykoside, die die Komponente (a) bilden, stellen bekannte nichtionische Tenside dar, die der Formel (I) folgen,

**R**^{**1**}**O-[G]**_{**p**} **(I)**

in der R¹ für einen Alkyl- und/oder Alkenylrest mit 4 bis 22 Kohlenstoffatomen, G für einen Zuckerrest mit 5 oder 6 Kohienstoffatomen und p für Zahlen von 1 bis 10 steht Sie können nach den einschlägigen Verfahren der präparativen organischen Chemie erhalten werden. Stellvertretend für das umfangreiche Schrifttum sei hier auf die Übersichtsarbeit von Biermann et al. in **Starch/Stärke 45, 281 (1993),** B.Salka in **Cosm.Toil. 108, 89 (1993)** sowie J.Kahre et al. in **SÖFW-Journal Heft 8, 598 (1995)** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestem oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich femer auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Des weiteren können sich die Glykoside auch von Glycerin oder Guerbetalkoholen mit 16 bis 36 Kohlenstoffatomen ableiten. Bevorzugt sind Alkyloligoglucoside auf Basis von gehärtetem C_{12/14}-Kokosalkohol mit einem DP von 1 bis 3.

### Polyolpoly-12-hydroxystearate

Bei den Polyolpoly-12-hydroxystearaten, die die Komponente (b) bilden, handelt es sich um bekannte Stoffe, die beispielsweise unter den Marke "Dehymuls® PGPH" von der Henkel KGaA, Düsseldorf/FRG vertrieben werden. In diesem Zusammenhang sei femer auf die Intemationale Patentanmeldung **WO 95/34528** (Henkel) verwiesen. Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
(a) Glycerin und Polyglycerin;
(b) Alkylenglycole, wie beispielsweise Ethylenglycol, Diethylenglycol, Propylenglycol;
(c) Methylolverbindungen, wie insbesondere Trimethylolethan, Trimethylolpropan, Trimethylolbutan, Pentaerythrit und Dipentaerythrit;
(d) Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
(e) Zuckeralkohole mit 5 bis 12 Kohlenstoffatomen, wie beispielsweise Sorbit oder Mannit;
(f) Zucker mit 5 bis 12 Kohlenstoffatomen, wie beispielsweise Glucose oder Saccharose;
(g) Aminozucker, wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammem angegeben sind die bevorzugten Bereiche):

| | |
|---|---|
| Glycerine | 5 bis 35(15 bis 30) Gew.-% |
| Diglycerine | 15 bis 40 (20 bis 32) Gew.-% |
| Triglycerine | 10 bis 35 (15 bis 25) Gew.-% |
| Tetraglycerine | 5 bis 20(8 bis 15) Gew.-% |
| Pentaglycerine | 2 bis 10(3 bis 8) Gew.-% |
| Oligoglycerine | ad 100 Gew.-% |

Die Komponenten (a) und (b) können im Gewichtsverhältnis 10 : 90 bis 90 : 10, vorzugsweise 25 : 75 bis 75 : 25 und insbesondere 40 : 60 bis 60 : 40 eingesetzt werden. Besonders bevorzugt ist eine Mischung aus Alkylglucosiden und Polyglycerinpoly-12-hydroxystearat im Gewichtsverhältnis 1 : 1, die unter der Marke Eumulgin® VL75 im Handel erhältlich ist. Der Anteil der Lösungsvermittler an den Endformulierungen kann 1 bis 25, vorzugsweise 5 bis 15 Gew.-% betragen.

### Etherische Öle

Zu den Stoffen mit ölähnlichen Eigenschaften, die sich wie oben beschrieben solubilisieren lassen, zählen beispielsweise die etherischen Öle. Hierunter versteht der Fachmann duftende Stoffe, die durch physikalische, vorzugsweise destillative Verfahrensschritte aus Pflanzen, Pflanzenteilen oder auch Gewürzen gewonnen werden. Etherische Öle lassen sich von den bekannten fetten Ölen durch einen einfachen Test unterscheiden: auf Papier hinterlassen sie keine Fettflecken. Es handelt sich bei diesen Stoffen um komplexe Gemische von teilweise leicht flüchtigen Alkoholen, Aldehyden, Ketonen, Estem, Lactonen, Schwefel- und Stickstoffhaltigen Verbindungen sowie Kohlenwasserstoffen; die Mehrzahl der geruchsbestimmenden Bestandteile der etherischen Öle stellen dabei Terpene und Sequiterpene dar. Typische Beispiele für geeignete etherische Öle sind die Extrakte von Blüten (Lavendel, Rosen, Jasmin, Neroli), Stengeln und Blättem (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedem-, Rosenholz), Kräutem und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Als synthetische bzw. halbsynthetische etherische Öle kommen Ambroxan, Eugenol, isoeugenol, Citronellal, Hydroxycitronellal, Geraniol, Citronellol, Geranylacetat, Citral, lonon und Methylionon in Betracht.

### Vitamine

Neben den etherischen Ölen können auch Vitamine mit Hilfe der Mischungen aus Alkylglykosiden und Polyglycerinestem solubilisiert werden. Typische Beispiele sind Vitamine der A-Gruppe (Retinol, 3,4-Dehydroretinol), der B-Gruppe (Thiaminchlorid-Hydrochlorid, Riboflavin, Pyridoxin, Pyridoxamin, Pyridoxal, Cyanocabalmin, Biotin, Folsäure, Nicotinsäure, Nicotinsäureamid, Pantothensäure), der C-Gruppe (Ascorbinsäure), der D-Gruppe (Calciferol, Ergocalciferol, Colecaliferol), sowie der E-Gruppe (Tocopherole, Tocopherolacetate). Die etherischen Öle bzw. Vitamine können in Mengen von 0,1 bis 10, vorzugsweise 0,5 bis 1 Gew.-% - bezogen auf die Solubilisate - eingesetzt werden.

### Hilfs- und Zusatzstoffe

Die Solubilisate können rein wäßriger Natur sein, es kommen aber auch wäßrig-alkoholische Anbietungsformen in Frage, die unter Verwendung von Ethanol oder Isopropylalkohol hergestellt werden können. Das Mischungsverhältnis mit Wasser ist an sich unkritisch, 60 bis 70 Gew.-%ige alkoholische Lösungen sind jedoch bevorzugt und lassen sich dann beispielsweise zusammen mit geeigneten Pflegestoffen als Lotionen zum Tränken von Erfrischungstüchem, sowie als Deoformulierungen, After Shaves, Eau de Toilettes und dergleichen verwenden.

Die Solubilisate können femer als weitere Hilfs- und Zusatzstoffe milde Tenside, Ölkörper, Überfettungsmittel, Stabilisatoren, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestem mit Polyolen, Siliconöle und/oder aliphatische bzw. naphthenische Kohlenwasserstoffe in Betracht.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose-und Hydroxyethylcellulose, femer höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinyl-imidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vemetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ lsobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiete für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinyl-pyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäureisopentylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4-tert.Butylphenyl)-3(4'-methoxyphenyl)propan-1,3-dionoder 1-Phenyl-3-(4'-isopropylphenyl)propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aiuminiumoxid, Ceroxid, Zirkoniumoxid, Silicate (Talk), Bariumsulfat und Zinkstearat Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopheroie (Vitamin E) und Ascorbinsäure (Vitamin C). Weitere geeignete UV-Lichtschutzfilter sind der Übersicht von P.Finkel in **SÖFW-Journal 122, 543 (1996)** zu entnehmen.

Zur Verbesserung des Fließverhaltens können femer **Hydrotrope**, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als Farbstoffe können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

### Beispiele

Lavandinöl wurde 1 Gew.-%ig in wäßrigen Lösungen verschiedener Solubilisatoren eingerührt. Das Ergebnis ist in Tabelle 1 zusammengefaßt Beispiel 1 ist erfindungsgemäß, die Beispiele V1 und V2 dienen zum Vergleich.

**Tabelle 1**

| **Solubilisierung von Lavendelöl (Mengenangaben als Gew.-%)** | | | |
|---|---|---|---|
| **Zusammensetzung / Beurteilung** | **1** | **V1** | **V2** |
| Lavandinöl | 1,0 | 1,0 | 1,0 |
| Polyglyceryl-2 Dipolyhydroxystearate (and) Laurylglucoside* | 5,0 | - | - |
| Polyglyceryl-2 Dipolyhydroxystearate | - | 5,0 | - |
| Laurylglucoside | - | - | 5,0 |
| Wasser | ad 100 | | |
| ***Beurteilung*** | klares Solubilisat | separiert, trüb | Separiert, trüb |

| | | | |
|---|---|---|---|
| *) Gewichtsverhältnis 1:1 | | | |

In Tabelle 2 sind weitere Beispielformulierungen angegeben. Dabei stellen die Rezepturen (A) bis (C) Tränkflüssigkeiten für Reinigungsfücher, (D) eine Deoformulierung, (E) ein Eau de Toilette und (F) ein After Shave dar.

**Tabelle 2**

| **Beispielformulierungen** | | | | | | |
|---|---|---|---|---|---|---|
| **Zusammensetzung** | **A** | **B** | **C** | **D** | **E** | **F** |
| Polyglyceryl-2 Dipolyhydroxystearate (and) Laurylglucoside | 9,7 | 12,5 | 9,0 | 10,0 | 14,8 | 12,5 |
| Triethyl Citrate | - | - | - | 2,0 | - | - |
| Coco Glycerides | 0,5 | - | - | - | - | - |
| Lavandinöl | 0,3 | - | 0,5 | 1,0 | 5,0 | 2,0 |
| Bergamotteöl | - | 1,5 | - | - | - | - |
| Isoamyl p-Methoxycinnamate | - | - | - | - | - | 0,2 |
| Dimethicon Copolyol | - | - | 1,0 | 1,0 | - | 1,0 |
| Ethanol | - | - | - | 10,0 | - | 10,0 |
| Methylparaben | 0,2 | 0,2 | 0,2 | 0,2 | 0,2 | 0,2 |
| Carbomer (+ KOH, pH 5,5) | - | - | 0,05 | - | - | - |
| Panthenol | 1,0 | 1,0 | 1,0 | - | - | 1,0 |
| Bisabolol | - | 0,2 | 0,2 | - | - | - |
| Wasser | ad 100 | | | | | |

## Patentansprüche

1. Verwendung von Detergensgemischen, enthaltend
(a) Alkyl- und/oder Alkenyloligoglykoside und
(b) Polyolpolyhydroxy-12-stearate
als Lösungsvermittler zur Herstellung von klaren Solubilisaten.

2. Verwendung nach Anspruch 1, **dadurch gekennzeichnet, daß** man als Komponente (a) Alkyl- und Alkenyloligoglykoside der Formel (I) einsetzt,
**R**^{**1**}**O-[G]**_{**p**} **(I)**
in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

3. Verwendung nach den Ansprüchen 1 und 2, **dadurch gekennzeichnet, daß** man als Komponente (b) Polyglycerinpoly-12-hydroxystearate einsetzt.

4. Verwendung nach den Ansprüchen 1 bis 3, **dadurch gekennzeichnet, daß** man die Komponenten (a) und (b) im Gewichtsverhältnis 10:90 bis 90:10 einsetzt.

5. Verwendung nach den Ansprüchen 1 bis 4, **dadurch gekennzeichnet, daß** man die Lösungsvermittler in Mengen von 1 bis 25 Gew.-% - bezogen auf die Emulsionen - einsetzt.

6. Verwendung nach den Ansprüchen 1 bis 5, **dadurch gekennzeichnet, daß** man etherische Öle und/oder Vitamine solubilisiert.

7. Verwendung nach den Ansprüchen 1 bis 6, **dadurch gekennzeichnet, daß** man die etherischen Öle bzw. Vitamine in Mengen von 0,1 bis 10 Gew.-% - bezogen auf die Solubilisate - einsetzt.

## Claims

1. The use of detergent mixtures containing
(a) alkyl and/or alkenyl oligoglycosides and
(b) polyolpolyhydroxy-12-stearates
as solubilizers for the production of clear solubilizates.

2. The use claimed in claim 1, **characterized in that** alkyl and alkenyl oligoglycosides corresponding to formula (I):
**R**^{**1**}**O-[G]**_{**p**} **(I)**
where R¹ is an alkyl and/or alkenyl group containing 4 to 22 carbon atoms, G is a sugar unit containing 5 or 6 carbon atoms and p is a number of 1 to 10, are used as component (a).

3. The use claimed in claims 1 and 2, **characterized in that** polyglycerol poly-12-hydroxystearates are used as component (b).

4. The use claimed in claims 1 to 3, **characterized in that** components (a) and (b) are used in a ratio by weight of 10:90 to 90:10.

5. The use claimed in claims 1 to 4, **characterized in that** the solubilizers are used in quantities of 1 to 25% by weight, based on the emulsions.

6. The use claimed in claims 1 to 5, **characterized in that** essential oils and/or vitamins are solubilized.

7. The use claimed in claims 1 to 6, **characterized in that** the essential oils and vitamins are used in quantities of 0.1 to 10% by weight, based on the solubilizates.

## Revendications

1. Utilisation de mélanges détergents, contenant :
a) des alkyl- et/ou alkényloligoglycosides, et
b) des polyhydroxy-12 stéarates de polyol
en tant qu'adjuvants de solubilisation en vue de la production de produits de solubilisation limpides.

2. Utilisation suivant la revendication 1,
**caractérisée en ce qu'**
on met en oeuvre comme composants a) des alkyl et des alkényloligoglycosides de formule (I)
R¹O-[G]ₚ (I)
dans laquelle R¹ représente un reste alkyle et/ou alkényle ayant 4 à 22 atomes de carbone, G représente un sucre ayant 5 ou 6 atomes de carbone et p représente des nombres allant de 1 et 10.

3. Utilisation selon l'une quelconque des revendications 1 et 2,
**caractérisée en ce qu'**
on met en oeuvre comme composants (b) des poly 12-hydroxystéarates de polyglycérine.

4. Utilisation selon l'une quelconque des revendications 1 à 3,
**caractérisée en ce qu'**
on met en oeuvre les composants a) et b) dans un rapport en poids allant de 10:90 et 90:10.

5. Utilisation selon l'une quelconque des revendications 1 à 4,
**caractérisée en ce qu'**
on met en oeuvre l'adjuvant de solubilisation en quantités allant de 1 à 25 % en poids rapporté aux émulsions.

6. Utilisation selon l'une quelconque des revendications 1 à 5,
**caractérisée en ce qu'**
on solubilise des huiles essentielles et/ou des vitamines.

7. Utilisation selon l'une quelconque des revendications 1 à 6,
**caractérisée en ce qu'**
on met en oeuvre les huiles essentielles ou les vitamines en quantités allant de 0,1 à 10 % en poids, rapporté aux produits de solubilisation.
